**Europäisches Patentamt**

**European Patent Office**

**Office européen des brevets**

(19)

(11) Veröffentlichungsnummer: **0 116 259**
**A1**

(12) # EUROPÄISCHE PATENTANMELDUNG

(21) Anmeldenummer: **83810557.5**

(22) Anmeldetag: **01.12.83**

(51) Int. Cl.³: **A 61 M 1/03**

(30) Priorität: **10.02.83 CH 768/83**

(43) Veröffentlichungstag der Anmeldung:
**22.08.84 Patentblatt 84/34**

(84) Benannte Vertragsstaaten:
**DE FR GB**

(71) Anmelder: **Laboratorium Dr. G. Bichsel AG**
**Bahnhofstrasse 5a**
**CH-3800 Interlaken(CH)**

(72) Erfinder: **Bichsel, Guido, Dr.**
**Bahnhofstrasse 7**
**CH-3800 Interlaken(CH)**

(74) Vertreter: **Gasser, François W.**
**Hirschengraben 10 Postfach 1555**
**CH-3001 Bern(CH)**

(54) **Beutelsystem für die Durchführung der ambulanten Peritonealdialyse und Verfahren zu seiner Anwendung.**

(57) Das im ersten Beutel (1) enthaltene frische Dialysat wird erst dann in die Bauchhöhle (8) des Patienten eingebracht, wenn die verbrauchte Dialysatlösung aus diesem in einen zweiten Beutel (7) abgeleitet und die Schläuche (9;6) mittels des im vom ersten Beutel (1) zum Y-Stück (4) führenden Desinfektionsmittel gereinigt worden ist. Dadurch wird das Peritonitisrisiko sehr klein und der Dialysewechsel sehr einfach.

Fig. 2

EP 0 116 259 A1

BEUTELSYSTEM FUER DIE DURCHFUEHRUNG DER AMBULANTEN
PERITONEALDIALYSE UND VERFAHREN ZU SEINER ANWENDUNG

Die vorliegende Erfindung betrifft einerseits ein Beutelsystem für die Durchführung der ambulanten Peritonealdialyse und andererseits das Verfahren zu seiner Anwendung.

Bei niereninsuffizienten Patienten wird die Peritonealdialyse als chronische Entgiftungsmethode angewendet.
Sie setzt einen offenen Zugang zur Bauchhöhle voraus, was
mittels eines Tenckhoff-Katheters erreicht wird. Um der
dadurch geschaffenen Gefahr von sekundären Infektionen,
Peritonitis, zu begegnen und die Dialyse selber einfacher
und risikoloser zu gestalten, als dies bisher der Fall
war, war es notwendig, ein neues Beutelsystem zu entwikkeln, wie es im kennzeichnenden Teil des Patentanspruches
1 definiert ist. Verbunden mit dem Verfahren zu seiner
Anwendung nach Patentanspruch 5 lässt sich die Peritonitisrate gegenüber herkömmlichen Peritonealdialyse-Systemen deutlich senken.

Im folgenden wird das erfindungsgemässe Beutelsystem sowie das Verfahren zu seiner Anwendung anhand der Zeichnung erläutert. In dieser zeigt

Fig. 1 das Beutelsystem mit seinen einzelnen Komponenten und

Fig. 2 schematisch den Ablauf eines Dialysatwechsels mit dem System nach Fig. 1.

Das Beutelsystem gemäss der Erfindung besteht im wesentlichen aus zwei Beuteln, die über ein Y-Verbindungsstück sowohl miteinander als auch mit einem in die Bauchhöhle des Patienten reichenden Tenckhoff-Katheter verbindbar sind. Im einen Beutel kann die frische Dialyselösung gelagert werden, wogegen das aus der Bauchhöhle zu entnehmende Dialysat in den zweiten Beutel fliessen kann. Eine vor dem Ansetzen der Dialyse im zum ersten Beutel führenden Schlauch gelagerte Desinfektionsflüssigkeit sorgt für absolute Sauberkeit des Systems, bevor die neue Dialyselösung in die Bauchhöhle geleitet wird.

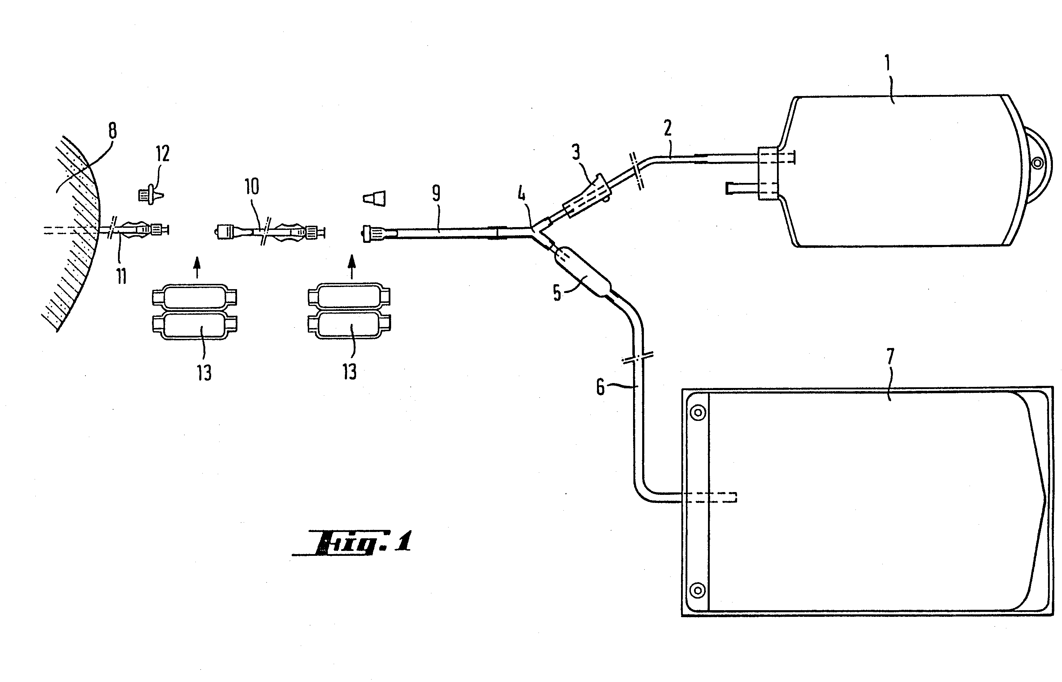

In Fig. 1 ist das Beutelsystem schematisch dargestellt. Der die frische Dialyselösung aufnehmende erste Beutel ist mit der Referenznummer 1 bezeichnet. Er ist über einen ersten Schlauch 2, der, weil er eine genügende Menge von Desinfektionsmittel aufnehmen muss, vorteilhafterweise eine Länge von ca. 1,5 m aufweist und einen bekannten Rollschieber 3 trägt, mit einem Y-Stück 4 verbunden. Letzteres ist seinerseits über eine Tropfkammer 5 und einen zweiten Schlauch 6 mit einem zweiten Beutel 7 verbunden, der das Dialysat aus der Bauchhöhle 8 des Patienten, sowie nach der Desinfektion des Systems die Desinfektionslösung aufzunehmen hat. Der dritte Schenkel des Y-Stückes 4 ist über einen dritten Schlauch 9 und ein Katheter-Verlängerungsstück 10 mit dem dem Patienten implantierten Tenckhoff-Katheter 11 verbindbar. Selbstverständlicherweise werden für alle Verbindungen zwischen den Beuteln und Schläuchen und anderen Elementen vorteilhafterweise Elemente des Luer-Lock-Schliess-Systems und Metallkonnektoren verwendet, die in der Medizin seit langem eingeführt sind. Um unerwünschte Infektionskeime am Eindringen in das Peritoneum zu hindern, ist ein Sicherheitsverschluss 12 vorgesehen, der den Tenckhoff-Katheter 11 völlig dicht abschliesst, wenn nicht das erfindungsgemässe Beutelsystem daran angeschlossen ist. Ferner werden während der Dialyse die beiden Verbindungen zwischen Katheter 11 und dem Verlängerungsstück 10, sowie diesem und dem dritten Schlauch 9, vorteilhafterweise durch Protektoren 13, die mit einer Betadine-getränkten Einlage versehen sind, geschützt.

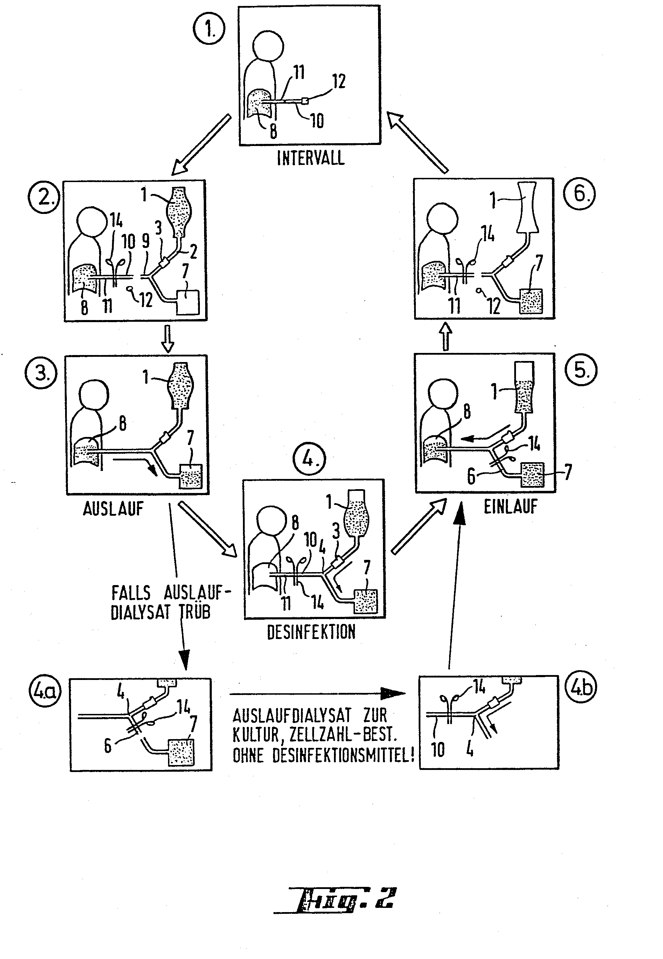

Das Verfahren zur Anwendung des erfindungsgemässen Beutelsystems geht schematisch aus Fig. 2 hervor, wo die verschiedenen Verfahrensschritte in je einer Position dargestellt sind. Position ① zeigt die übliche Situation für den Patienten, dem der Tenckhoff-Katheter 11 in die Bauchhöhle 8 eingesetzt ist. Das Verlängerungsstück 10 ist, sofern überhaupt vorhanden, vorteilhafterweise fest mit dem Katheter 11 verbunden. Entweder der Katheter oder das Verlängerungsstück 10 ist mit einem Sicherheitsverschluss 12 verschlossen. Aus Pos. ② ersieht man die Vorbereitung für die Dialyse. Der Katheter 11 oder das Verlängerungsstück 10 wird mittels einer Schlauchklemme 14 abgeklemmt, worauf der Sicherheitsverschluss 12 entfernt und an seiner Stelle der dritte Schlauch 9 des Beutelsystems angeschlossen wird. Lezteres weist im ersten Beutel 1 die frische Dialyselösung auf, wogegen der zweite Beutel 7 leer ist. Im ersten Schlauch 2, vor dem Rollschieber 3, befindet sich eine Desinfektionsflüssigkeit.

Nach Abschluss des Beutelsystems wird, wie in Pos.③ dargestellt, das in der Bauchhöhle 8 des Patienten vorhandene Dialysat in den zweiten Beutel 7 ausfliessen gelassen. Im Normalfall, wenn das Auslaufdialysat klar ist, wird nach vollständiger Entleerung der Bauchhöhle 8 die Verlängerung 10 wieder mittels einer Schlauchklemme 14 verschlossen, bevor der Rollschieber 3 so lange geöffnet wird (Pos.④) , dass die Desinfektionsflüssigkeit durch das Y-Stück 4 in den zweiten Beutel 7 abfliessen und dabei letzteres desinfizieren kann. Dann wird, wie in Pos.⑤ dargestellt, die Schlauchklemme 14 an den zweiten Schlauch 6 gelegt, so dass der zweite Beutel 7 verschlossen und der Weg zur Bauchhöhle 8 wieder frei ist. Jetzt kann das frische Dialysat aus dem ersten Beutel 1 in die Bauchhöhle 8 fliessen, bevor wiederum der Katheter 11 mittels der Schlauchklemme 14 verschlossen wird (Pos⑥), um das Entfernen des Beutelsystems 1, 7 zu ermöglichen. Nachdem das Verlängerungsstück 10 wiederum durch den Sicherheitsverschluss 12 verschlossen worden ist, kann auch die Schlauchklemme 14 wieder entfernt werden und der Patient kann sich wieder bis zur nächsten Dialyse frei bewegen (Pos.①).

Sollte festgetellt werden, dass das Auslaufdialysat bei Pos.③ trüb ist, ist gemäss den Pos. ④a und ④b vorzugehen. Dabei muss, wie aus Pos. ④a ersichtlich, während des Auslaufens des sich in der Bauchhöhle 8 des Patienten befindlichen Dialysats zwischenhinein der zweite Schlauch 6 abgeklemmt werden, so dass eine Probe entnommen werden kann, bevor das Desinfektionsmittel wie in Pos. ④b gezeigt, das Y-Stück 4 reinigt.

Der dank dem erfindungsgemässen Beutelsystem sehr einfach gewordene Dialysatwechsel kann vom Patienten zuhause durchgeführt werden und erleichtert ihm daher das Leben mit seinem Leiden ganz wesentlich. Durch die Verwendung eines geschlossenen Systems mit Desinfektionslösung kann zudem die Infektionsgefahr wesentlich vermindert werden.

Der Fachmann erkennt leicht, dass das hiervor beschriebene und in der Zeichnung dargestellte Beutelsystem in Einzelheiten anders ausgeführt werden kann. So ist es ohne weiteres möglich, das Y-Stück 4 durch ein anderes Element zu ersetzen. Die Tropfkammer 5 kann gegebenenfalls weggelassen werden, ohne dass dadurch die Funktionsweise des Systems beeinträchtigt würde. Auch sind andere als Luer-Lock-Verbindungsstücke verwendbar. Das Verlängerungsstück 10 ist auch nicht unbedingt erforderlich.

0116259

- 6 -

PATENTANSPRUECHE

1. Beutelsystem für die Durchführung der ambulanten Peritonealdialyse, dadurch gekennzeichnet, dass es zwei über ein Verbindungsstück (4) miteinander verbundene Beutel (1;7) aufweist und mit einem Anschlussschlauch (9) mit einem in die Bauchhöhle (8) des Patienten eingelegten Katheter (11) verbindbar ist.

2. Beutelsystem nach Anspruch 1, dadurch gekennzeichnet, dass ein Beutel (1) mit frischer Dialyselösung gefüllt ist, wogegen der zweite Beutel (7) für die Aufnahme des gebrauchten, aus der Bauchhöhle (8) des Patienten auslaufenden Dialysats dient.

3. Beutelsystem nach Anspruch 1, dadurch gekennzeichnet, dass ein den ersten Beutel (1) mit dem Verbindungsstück (4) verbindender erster Schlauch (2) mittels eines Rollschiebers (3) verschliessbar ist und mit Desinfektionslösung gefüllt ist.

4. Beutelsystem nach Anspruch 1, dadurch gekennzeichnet, dass ein den zweiten Beutel (7) mit dem Verbindungsstück (4) verbindender zweiter Schlauch (6) eine Tropfkammer (5) aufweist.

5. Verfahren zur Anwendung des Beutelsystems nach den Ansprüchen 1 bis 4, dadurch gekennzeichnet, dass es mittels Luer-Lock-Konnektoren an einen in die Bauchhöhle (8) eines Patienten hineinreichenden Katheter (18) angeschlossen wird, worauf das sich in der Bauchhöhle befindliche Dialysat in den zweiten Beutel (7) fliessen gelassen wird, worauf die Desinfektionslösung aus dem ersten Schlauch (2) in den zweiten Beutel (7) fliessen gelassen wird, bevor das frische Dialysat
aus dem ersten Beutel (1) in die Bauchhöhle (18) des
Patienten fliessen gelassen wird.

6. Verfahren nach Anspruch 5, dadurch gekennzeichnet,
dass der zweite und dritte Schlauch (6;9) nach Bedarf
mit einer Schlauchklemme (14) verschlossen wird.

**Fig. 1**

Fig. 2

# EUROPÄISCHER RECHERCHENBERICHT

EP  83 81 0557

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| X | US-A-4 306 976  (BIEFFE)<br>*  Insgesamt  und  insbesondere Figuren  1,2;  Spalte  1,  Zeilen 7-19, 47-57; Spalte 3, Zeilen 1-7 * | 1,2 | A 61 M   1/03 |
| Y | | 3,5 | |
| A | | 4 | |
| | --- | | |
| Y | EP-A-0 049 525  (SIS-TER)<br>*  Insgesamt  und  insbesondere Figuren   1,2;   Bezugszeichten 8,17,19;  Seite  5, Zeilen 20-22; Seite 7, Zeilen 8-12, 17-18 * | 3 | |
| | --- | | |
| Y | GB-A-2 059 776  (MILLIPORE)<br>* Figur 1, Bezugszeichen 24,30 * | 3 | RECHERCHIERTE SACHGEBIETE (Int. Cl. ³) |
| A | | 4 | A 61 M |
| | --- | | |
| Y | EP-A-0 062 148  (LAB. HAUSMANN)<br>* Seite 6, Zeilen 5-11 * | 5 | |
| | --- | | |
| Y | DE-A-2 809 303  (POPOVICH)<br>*  Insgesamt  und  insbesondere Figuren  2,4D; Seite 13, Zeile 26 -  Seite  14,  Zeile 2; Seite 14, Zeilen 5-7, 9-11; Seite 15, Zeile 14 - Seite 16, Zeile 2 * | 5 | |
| | ---          -/- | | |

Der vorliegende Recherchenbericht wurde für alle Patentansprüche erstellt.

| Recherchenort | Abschlußdatum der Recherche | Prüfer |
|---|---|---|
| DEN HAAG | 19-04-1984 | PETZUCH M. |

**Europäisches Patentamt**

# EUROPÄISCHER RECHERCHENBERICHT

EP 83 81 0557

| | **EINSCHLÄGIGE DOKUMENTE** | | Seite 2 |
|---|---|---|---|
| **Kategorie** | **Kennzeichnung des Dokuments mit Angabe, soweit erforderlich, der maßgeblichen Teile** | **Betrifft Anspruch** | **KLASSIFIKATION DER ANMELDUNG (Int. Cl. ³)** |
| X | FR-A-2 486 803 (DRAMTCHEV) <br> * Figur 1 und insbesondere Bezugszeichen 12,13 * | 1,2 | |
| A | | 6 | |
| | ----- | | |
| | | | **RECHERCHIERTE SACHGEBIETE (Int. Cl. ³)** |
| Der vorliegende Recherchenbericht wurde für alle Patentansprüche erstellt. | | | |

| **Recherchenort** <br> DEN HAAG | **Abschlußdatum der Recherche** <br> 19-04-1984 | **Prüfer** <br> PETZUCH M. |
|---|---|---|